# EUROPEAN PATENT APPLICATION

(11) **EP 4 098 744 A1**
(43) Date of publication of application: **07.12.2022**
(21) Application number: 21747077.2
(22) Date of filing: 29.01.2021
(51) Int. Cl.: C12N 15/09, C12N 15/10, C12Q 1/6813, C12Q 1/6876

(54) **METHOD FOR DETECTING OR QUANTIFYING NUCLEIC ACID**

(30) Priority: 31.01.2020 JP 2020014497
(71) Applicant: Sekisui Medical Co., Ltd., Tokyo 103-0027 (JP)
(72) Inventor: OOMORI Akane, Tokyo 103-0027 (JP); OSAWA Masako, Tokyo 103-0027 (JP)
(74) Representative: Hoffmann Eitle
(86) International application number: PCT/JP2021/003144
(87) International publication number: WO 2021/153709

(57) **Abstract**

An object of the present invention is to provide a more sensitive detection method and quantification method in a method for detecting a target nucleic acid using the PALSAR method. The present invention provides a method for recovering a target nucleic acid in a sample, comprising: (i) a step of bringing a target nucleic acid in a sample, a capture probe, and an assist probe into contact with each other for hybridization, the capture probe containing (A) a nucleic acid probe, and (B) a solid phase or an adapter or linker flanking to a 3'-end or 5'-end nucleotide of the nucleic acid probe, the nucleic acid probe containing: a first base sequence; and a second base sequence complementary to a complete or partial sequence of the target nucleic acid, the assist probe containing a 6- to 9-mer sequence capable of flanking to the target nucleic acid and complementary to a complete or partial sequence of the first base sequence of the nucleic acid probe; and (ii) a step of recovering a hybridization product contained in the sample.

## Description

### TECHNICAL FIELD

The present invention relates to a highly sensitive and highly quantitative nucleic acid detection or quantification method.

### BACKGROUND ART

The PALSAR method is known as a technique for detecting target nucleic acids of DNA and RNA. In the principle of the PALSAR method, a set of signal amplification probes (sometimes referred to as self-aggregation probes in this description) consisting of three complementary regions prepared in advance is used, and the probes are labeled, and since the probes repeat a self-aggregation reaction through hybridization to form a reticulated large DNA aggregate (sometimes referred to as oligonucleotide polymer in the present specification), which is used as an amplification signal. This large aggregate of DNA is bound to DNA or RNA to be detected, so that the amplified signal is captured to detect and quantify the DNA or RNA to be detected in this method.

The method of Patent Document 1 is known as a method for detecting a target nucleic acid using the PALSAR method.

In this method, a target nucleic acid in a sample is detected by using signal amplification probes used in the PALSAR method, a first nucleic acid probe having a sequence complementary to the target nucleic acid, and a second nucleic acid probe having a sequence complementary to the first nucleic acid probe, capable of binding to the first nucleic acid probe in a state of flanking to the target nucleic acid, and capable of binding to at least one of the signal amplification probes. In this method, when the target nucleic acid is present in the sample, the oligonucleotide polymer formed by the self-aggregation reaction of the signal amplification probes is detected in a state of being bound to a complex containing the target nucleic acid, the first nucleic acid probe, and the second nucleic acid probe, and when the target nucleic acid is not present in the sample, the second nucleic acid probe dissociates from the first nucleic acid probe, and the oligonucleotide polymer bound to the complex is not detected. However, when the present inventors reproduced this method, a problem was found that a signal value was high even when the target nucleic acid was not present, which lowers the so-called S/N ratio (signal-to-noise value).

### CITATION LIST

### PATENT LITERATURE

Patent Document 1: Japanese Laid-Open Patent Publication No. 2012-70635

### SUMMARY OF INVENTION

### TECHNICAL PROBLEM

In the method for detecting the target nucleic acid using the PALSAR method, a more sensitive detection method and quantification method have been required.

### SOLUTION TO PROBLEM

To solve the problem, various studies were conducted on the first nucleic acid probe, the second nucleic acid probe, reaction conditions, etc., we surprisingly found that by changing a relationship between the length of the second nucleic acid probe flanking to the target nucleic acid (hereinafter, a portion of an assist probe of the present invention complementary to the first base sequence) and the length of the first base sequence of the first nucleic acid probe complementary thereto (hereinafter, corresponding to a capture probe of the present invention), the background can be suppressed and the S/N ratio can be improved to further increase the detection sensitivity, thereby completing the present invention.

The present invention has the following configuration.
<1> A method for recovering a target nucleic acid in a sample, comprising:
   (i) a step of bringing a target nucleic acid in a sample, a capture probe, and an assist probe into contact with each other for hybridization,
      the capture probe containing
         (A) a nucleic acid probe, and
         (B) a solid phase or an adapter or linker flanking to a 3'-end or 5'-end nucleotide of the nucleic acid probe,
      the nucleic acid probe containing:
         a first base sequence; and
         a second base sequence complementary to a complete or partial sequence of the target nucleic acid,
      the assist probe containing a 6- to 9-mer sequence capable of flanking to the target nucleic acid and complementary to a complete or partial sequence of the first base sequence of the nucleic acid probe; and
   (ii) a step of recovering a hybridization product contained in the sample.
<2> A method for detecting a target nucleic acid in a sample, comprising:
   (i) a step of bringing a target nucleic acid in a sample, a capture probe, and an assist probe into contact with each other for hybridization,
      the capture probe containing
         (A) a nucleic acid probe, and
         (B) a solid phase or an adapter or linker flanking to a 3'-end or 5'-end nucleotide of the nucleic acid probe,
      the nucleic acid probe containing:
         a first base sequence; and
         a second base sequence complementary to a complete or partial sequence of the target nucleic acid,
      the assist probe containing a 6- to 9-mer sequence capable of flanking to the target nucleic acid and complementary to a complete or partial sequence of the first base sequence of the nucleic acid probe;
   (ii) a step of recovering a hybridization product contained in the sample; and
   (iii) a step of detecting the recovered hybridization product.
<3> A method for detecting a target nucleic acid in a sample by forming a complex with a pair of probes capable of self-aggregation consisting of first and second oligonucleotides, comprising:
   (i) a step of bringing a target nucleic acid in a sample, a capture probe, and an assist probe into contact with each other for hybridization,
      the capture probe containing
         (A) a nucleic acid probe, and
         (B) a solid phase or an adapter or linker flanking to a 3'-end or 5'-end nucleotide of the nucleic acid probe,
      the nucleic acid probe containing:
         a first base sequence; and
         a second base sequence complementary to a complete or partial sequence of the target nucleic acid,
      the assist probe containing a 6- to 9-mer sequence capable of flanking to the target nucleic acid and complementary to a complete or partial sequence of the first base sequence of the nucleic acid probe, and a complete or partial sequence of at least one of the first and second oligonucleotides;
   (ii) a step of bringing the first and second oligonucleotides into contact with the hybridization product to form a polymer complex between the hybridization product and an oligonucleotide polymer formed by self-aggregation of the first and second oligonucleotides; and
   (iii) a step of detecting the polymer complex.
<4> The method according to any one of <1> to <3>, wherein the step of bringing a target nucleic acid in a sample, a capture probe, and an assist probe into contact with each other for hybridization includes two stages of hybridization steps, which are a first hybridization step of bringing the target nucleic acid into contact with the capture probe, and a second hybridization step of bringing a first hybridization product into contact with the assist probe.
<5> The method according to <4>, wherein the two stages of hybridization steps are steps performed at the same time.
<6> The method of any of <3> to <5>, wherein the assist probe contains a 6- to 9-mer sequence capable of flanking to the target nucleic acid and complementary to the complete sequence of the first base sequence of the nucleic acid probe, and a complete or partial sequence of at least one of the first and second oligonucleotides.
<7> The method according to any one of <1> to <6>, wherein the assist probe is capable of flanking to the end of the target nucleic acid.
<8> The method according to any one of <1> to <7>, wherein the sample is a blood-derived component.
<9> The method according to any one of <1> to <8>, wherein the target nucleic acid is a chemically modified nucleic acid.
<10> The method according to <9>, wherein the chemically modified nucleic acid is LNA, BNA, phosphorothioate oligo, morpholino oligo, boranophosphate oligo, 2'-O-methylated RNA (2'-OMe), 2'-O-methoxyethylated RNA (2'-MOE), or 2'-F-RNA.
<11> A kit for detecting a target nucleic acid in a sample, comprising:
   (1) a capture probe for capturing a target nucleic acid,
      the capture probe containing
         (A) a nucleic acid probe, and
         (B) a solid phase or an adapter or linker flanking to a 3'-end or 5'-end nucleotide of the nucleic acid probe,
      the nucleic acid probe containing:
         a first base sequence; and
         a second base sequence complementary to a complete or partial sequence of the target nucleic acid;
   (2) a pair of probes capable of self-aggregation consisting of first and second oligonucleotides; and
   (3) an assist probe containing a 6- to 9-mer sequence capable of flanking to the target nucleic acid and complementary to a complete or partial sequence of the first base sequence of the nucleic acid probe, and a complete or partial sequence of at least one of the first and second oligonucleotides.
<12> The detection kit according to <11>, wherein the assist probe contains a 6- to 9-mer sequence capable of flanking to the target nucleic acid and complementary to the complete sequence of the first base sequence of the nucleic acid probe, and a complete or partial sequence of at least one of the first and second oligonucleotides.
<13> The detection kit according to <11> or <12>, wherein the assist probe is capable of flanking to the end of the target nucleic acid.

### ADVANTAGEOUS EFFECTS OF INVENTION

According to the present invention, when the target nucleic acid is not present, the assist probe does not hybridize with or, even if temporarily hybridizes, immediately dissociates from the capture probe, and when the target nucleic acid is present, the assist probe of the present invention flanks to the target nucleic acid and hybridizes with the capture probe, so that the target nucleic acid can reliably be recovered or detected.

### BRIEF DESCRIPTION OF DRAWINGS

[Fig. 1] Fig. 1 is a step schematic diagram showing an aspect of basic steps of the present invention.
[Fig. 2] Fig. 2 is a graph showing signal values and S/N ratios when a length of a sequence complementary to a first base sequence of a nucleic acid probe of an assist probe is 8 mer, 9 mer, and 10 mer, in the presence and absence of the target nucleic acid (Example 1).
[Fig. 3] Fig. 3 is a graph showing signal values and S/N ratios when the length of the first base sequence of the nucleic acid probe is 7 to 10 mer, and the length of the sequence complementary to the first base sequence of the nucleic acid probe of the assist probe is identical to the length of the first base sequence of the nucleic acid probe, in the presence and absence of the target nucleic acid (Example 2).
[Fig. 4] Fig. 4 is a graph showing signal values and S/N ratios when the length of the sequences complementary to the first base sequence of the nucleic acid probe of the assist probe is 5 mer, 6 mer, and 7 mer, in the presence and absence of the target nucleic acid (Example 3).

### DESCRIPTION OF EMBODIMENTS

### (Recovery Method)

A first aspect of the present invention is a method for recovering a nucleic acid to be targeted (hereinafter, sometimes simply referred to as a target nucleic acid). The present invention includes the following steps (i) and (ii):
(i) a step of bringing a target nucleic acid in a sample, a capture probe, and an assist probe into contact with each other for hybridization, the capture probe containing
   (A) a nucleic acid probe, and
   (B) a solid phase or an adapter or linker flanking to a 3'-end or 5'-end nucleotide of the nucleic acid probe,
      the nucleic acid probe containing:
         a first base sequence; and
         a second base sequence complementary to a complete or partial sequence of the target nucleic acid,
      the assist probe containing a 6- to 9-mer sequence capable of flanking to the target nucleic acid and complementary to a complete or partial sequence of the first base sequence of the nucleic acid probe; and
(ii) a step of recovering a hybridization product contained in the sample,
   wherein
   the hybridization step of (i) includes a first hybridization step of hybridizing the target nucleic acid and a second base sequence region of the capture probe, and a second hybridization step of hybridizing the assist probe with the hybridization product so that the assist probe flanks to the target nucleic acid, and the first base sequence region of the capture probe and the assist probe are hybridized. These two hybridization steps may be performed simultaneously or separately.

The step of recovering a hybridization product of (ii) is the same as the method for separating a self-aggregation probe described later. Therefore, in addition to centrifugation and suction filtration, when the solid phase is a plate, the target product is bound to the plate, so that only the target product can be recovered by removing a supernatant by suction or decanting.

### (Detection method)

A second aspect of the present invention is a method for detecting a target nucleic acid, comprising steps (i) to (iii). Steps (i) and (ii) are the same steps as in the first aspect, and step (iii) is a step of detecting the recovered hybridization product. In this case, a detection object is a labeling substance of the assist probe, and the labeling to the assist probe will be described later.

### (Detection by PALSAR Method)

A third aspect of the present invention is a method for detecting a target nucleic acid by a PALSAR method, comprising steps (i) to (iii). The hybridization step of (i) is the same step as the first aspect.

Step (ii) is a step of bringing a pair of probes capable of self-aggregation and consisting of first and second oligonucleotides into contact with the hybridization product of the capture probe, the target nucleic acid, and the assist probe obtained in step (i) to form a complex between the hybridization product and an oligonucleotide polymer formed by self-aggregation of the first and second oligonucleotides.

Step (iii) is a step of detecting the complex.

Steps (ii) and (iii) are a signal amplification step using a self-aggregation probe and a step of detecting an amplified signal, which is the so-called PALSAR method (PALSAR method).

Hybridization, specific steps of the PALSAR method, the capture probe, the assist probe, and the self-aggregation probe will be described later.

### (Target Nucleic Acid)

The target nucleic acid of the present invention can target any nucleic acid such as DNA and RNA. The present invention is suitable for detecting nucleic acid medicines, which have been attracting particular attention and being actively developed in recent years as new molecular-targeted drugs. Since almost all products of nucleic acid drugs use nucleic acids with some modification so as to control degradation in the body, the detection technique of the present invention is preferably applied. Examples thereof include a Gapmer structure antisense nucleic acid medicine known as an "artificial nucleic acid-DNA-artificial nucleic acid" structure. Chemical modification may be applied to any site of a base portion, a sugar portion, and a phosphate portion of the nucleic acid.

Specifically, the target nucleic acid to be detected of the present invention is a natural DNA or RNA, or a chemically modified DNA or RNA, and examples of the chemical modification include phosphorothioate modification (S-), 2'-F modification, 2'-O-Methyl (2'-OMe) modification, 2'-O-Methoxyethyl (2'-MOE) modification, morpholino modification, LNA modification, BNA^{COC} modification, BNA^{NC} modification, ENA modification, cEtBNA modification, etc.

Among these, DNA or RNA with the 3'-terminal nucleic acid chemically modified is preferable, and LNA, BNA, phosphorothioate oligo, morpholino oligo, boranophosphate oligo, and 2'-O-methylated RNA (2'-OMe), 2'-O-methoxyethylated RNA (2'-MOE), and 2'-F-RNA are further preferable, and LNA or BNA is even more preferable.

The target nucleic acid may be either single-stranded or double-stranded, and in the case of double-stranded, the nucleic acid is usually made single-stranded and used in the present invention.

### (Capture Probe)

In the present invention, the "capture probe" is a probe for capturing a target nucleic acid and contains (A) a nucleic acid probe and (B) a solid phase or an adapter or linker flanking to a 3'-end or 5'-end nucleotide of the nucleic acid probe.

### (Nucleic Acid Probe)

The (A) nucleic acid probe used in the present invention contains a first base sequence and a second base sequence complementary to a complete or partial sequence of the target nucleic acid at a position flanking to the first base sequence. In the nucleic acid probe, the first base sequence is arranged without an interval from the second base sequence.

For the solid phase of (B), for example, it is preferable to use insoluble fine particles, microbeads, fluorescent fine particles, magnetic particles, a microplate, a microarray, a slide glass, or a substrate such as an electrically conductive substrate.

The second base sequence preferably contains a sequence complementary to the complete length of the target nucleic acid or to a portion containing the 3' end or 5' end of the target nucleic acid, and the following cases of (a-1) and (a-2) are available.
(a-1) When the solid phase or the adapter or linker flanks to a 3'-terminal nucleotide, the nucleic acid probe contains a sequence complementary to the target nucleic acid in a portion containing the 3'-terminal nucleotide.
(a-2) When the solid phase or the adapter or linker flanks to a 5'-end nucleotide, the nucleic acid probe contains a sequence complementary to the target nucleic acid in a portion containing the 5'-end nucleotide.

The sequence complementary to a complete or partial sequence of the target nucleic acid may be identical at a level enabling hybridization with the sequence of the target nucleic acid.

The length (number of bases, mer) of the second base sequence of the nucleic acid probe may be any length as long as the probe can bind to the target nucleic acid and, for example, the lower limit is preferably bases of 4 mer or more, more preferably bases of 10 mer or more, further preferably bases of 15 mer or more. As the upper limit, bases of 1000 mer or less are preferable.

The first base sequence of the nucleic acid probe contains a sequence that can hybridize with the assist probe described later, and the length of the sequence (number of bases, mer) may be any length that can bind to the assist probe and may be 6 mer or more, preferably 6 to 20 mer, more preferably 6 to 10 mer, and further preferably 6 to 9 mer.

### (Assist Probe)

The assist probe of the present invention is a probe containing a 6- to 9-mer sequence capable of flanking to the target nucleic acid (capable of being placed next to the target nucleic acid, in contact with the target nucleic acid / capable of being located adjacent to the target nucleic acid) and complementary to a complete or partial sequence of the first base sequence of the nucleic acid probe.

A first aspect of the assist probe is a probe used in a method for forming and recovering or detecting a hybridization product of the target nucleic acid, the capture probe, and the assist probe, and containing a 6- to 9-mer sequence capable of flanking to the target nucleic acid and complementary to a complete or partial sequence of the first base sequence of the nucleic acid probe. In the case of 5 mer or less, the effect of flanking to the target nucleic acid is reduced, and even if hybridizing with the first base sequence region of the nucleic acid probe, the sequence is immediately detached, which is not preferable, and in the case of 10 mer or more, the sequence binds to the first base sequence region of the nucleic acid probe even in the absence of the target nucleic acid. Preferably, the assist probe is capable of flanking to the end of the target nucleic acid. Being capable of flanking means that nucleic acids can be arranged without an interval. The effect of flanking (flanking effect) is that since nucleic acids are arranged without an interval, even if the number of bases is short, the probe can hybridize with the complementary nucleic acid to form and maintain a complementary strand without dropping off. When the hybridization product of the target nucleic acid, the capture probe, and the assist probe (a [target nucleic acid]-[capture probe]-[assist probe] complex) is detected in the first aspect, the assist probe is labeled, and the labeling substance is described later.

A second aspect of the assist probe is a probe used in a method for detecting the hybridization product by the PALSAR method, containing a 6- to 9-mer sequence capable of flanking to the target nucleic acid and complementary to a complete or partial sequence of the first base sequence of the nucleic acid probe, and containing a complete or partial sequence of at least one of first and second oligonucleotides constituting the self-aggregation probe. Preferably, the assist probe contains a 6- to 9-mer sequence capable of flanking to the target nucleic acid and complementary to the complete sequence of the first base sequence of the nucleic acid probe. The length of the sequence complementary to the complete sequence of the first base sequence is more preferably 7 to 9 mer. The assist probe in the second aspect may or may not be labeled with a labeling substance. The assist probe is preferably capable of flanking to the end of the target nucleic acid.

### (Solid Phase)

Examples of the solid phase in the present invention include insoluble fine particles, microbeads, fluorescent fine particles, magnetic particles, a microplate, a microarray, a slide glass, or a substrate such as an electrically conductive substrate, preferably fluorescent fine particles, and more preferably, fluorescent beads, particularly preferably beads having a fluorescent substance on a surface. The "beads having a fluorescent substance on a surface" used in the present invention are not particularly limited as long as the beads have a fluorescent substance, and for example, MicroPlex^{™} Microspheres manufactured by Luminex can preferably be used. One type of beads can be used, or multiple types of beads can be used. By using multiple types of color-coded beads, a method for detecting or quantifying a nucleic acid of the present invention can easily be multiplexed.

The solid phase and the nucleic acid probe may directly be bound or may be bound via an adapter or a linker and is preferably bound via an adapter or a linker.

### (Adapter or Linker)

Examples of the "adapter or linker" used in the present invention include compounds having an amino group or a carboxyl group such as spacers such as biotin, Spacer 9, Spacer 12, Spacer 18, and Spacer C3, and the adapter or linker is preferably 5'-Amino-Modifier C12 (12-(4-Monomethoxytritylamino)dodecyl-1-[(2-cyanoethyl)-(N,N-diisopropyl)]-phosphoramidite), biotin, etc.

For example, in an embodiment where the bead has a carboxy group on its surface and the nucleic acid probe having an amino group compound added thereto binds to the carboxyl group on the bead surface via the amino group, the compound having an amino group is an example of the linker.

In the present invention, "flanking" of "the solid phase or the adapter or linker flanking to a 3'-end or 5'-end nucleotide of the nucleic acid probe" means that the solid phase, the adapter, or the linker is bound to the nucleotide, and the meaning of the binding will be described later.

### (Labeling Substance)

Suitable examples of the labeling substance in the present invention include radioactive isotopes, biotin, digoxigenin, fluorescent substances, luminescent substances, or dyes.

Specifically, the target nucleic acid can be detected by adding radioisotopes such as ¹²⁵I and ³²P, digoxygenin, fluorescent dyes such as Cyanin 5, luminescent or coloring substances such as acridinium ester, fluorescein for using luminescent or coloring substances such as dioxetane and fluorescent substance such as 4-methylumbelliferyl phosphate via alkaline phosphatase, etc., bound to an anti-fluorescein antibody, biotin etc. for using fluorescent, luminescent, or coloring substances bound to avidin, or a donor fluorescent dye and an acceptor fluorescent dye for using fluorescence resonance energy transfer (FRET).

### (Binding of Nucleic Acid Probe to Solid Phase)

Examples of binding of the nucleic acid probe to the solid phase include a method using chemical bonding, a method using biological interaction, a method using physical adsorption, etc. In the method using chemical bonding, for example, when a solid phase coated with a carboxyl group is used, a coupling reaction can be achieved with an amino group labeling an oligonucleotide. The method using biological interaction can utilize, for example, the binding force between streptavidin coating the solid phase and biotin modifying the nucleic acid probe. In the method using physical adsorption, for example, when a solid phase having a negative charge is used, the oligonucleotide can be electrostatically adsorbed to the solid phase by labeling with a substance having a positive charge such as an amino group.

### (Contact)

As used herein, the term "bringing into contact" or "contacting step" means that one substance and another substance are placed in proximity to each other so as to form a chemical bond such as a covalent bond, an ionic bond, a metal bond, or a non-covalent bond between these substances. In one aspect of the present invention, "bringing" one substance "into contact with" another means mixing a solution containing one substance with a solution containing another substance.

The contacting step between the target nucleic acid in the sample and the capture probe is performed by keeping the temperature at 30 to 70 °C for 0.2 to 30 hours, preferably 35 to 65 °C for 0.5 to 24 hours. The contacting step between the [target nucleic acid]-[capture probe] complex in the sample and the assist probe is performed by keeping the temperature at 30 to 70 °C for 0.2 to 30 hours, preferably at 35 to 65 °C for 0.5 to 24 hours. The contacting step between the [target nucleic acid]-[capture probe]-[assist probe] complex in the sample and the first and second oligonucleotides is performed by keeping the temperature at 28 to 70 °C for 0.2 to 3 hours, preferably 30 to 54 °C for 0.5 to 2 hours.

The contact between the target nucleic acid in the sample and the capture probe means that the contact is performed under the condition enabling hybridization of corresponding bases of complementary sequences of the target nucleic acid and the nucleic acid probe. Similarly, the contact between the capture probe and the assist probe, the contact between a pair of probes capable of self-aggregation and the assist probe, and the contact between the paired self-aggregation probes are also performed under conditions enabling the hybridization of the corresponding bases.

### (Self-Aggregation)

As used herein, the term "self-aggregation" refers to a state in which multiple first oligonucleotides form a complex through hybridization with a second oligonucleotide, and a state in which multiple second oligonucleotides form a complex through hybridization with a first oligonucleotide.

### (Pair of Probes Capable of Self-Aggregation)

The pair of probes "capable of self-aggregation" used in the method of the present invention refers to oligonucleotides having a complementary base sequence region in which the first oligonucleotide and the second oligonucleotide are capable of hybridizing with each other, and capable of forming an oligonucleotide polymer through a self-aggregating reaction. Preferably, at least one of the first or second oligonucleotides is labeled with a labeling substance. As used herein, "capable of hybridizing" means, in one embodiment, being completely complementary in the complementary base sequence region. In another embodiment, this means being completely complementary in the complementary base sequence region except for one or two mismatches.

The pair of probes capable of self-aggregation can be labeled with a labeling substance for detection in advance. Suitable examples of such labeling substances include radioisotopes, biotin, digoxigenin, fluorescent substances, luminescent substances, or dyes. Specifically, the target nucleic acid can be detected by adding radioisotopes such as ¹²⁵I and ³²P, digoxygenin, fluorescent dyes such as Cyanin 5, luminescent or coloring substances such as acridinium ester, fluorescein for using luminescent or coloring substances such as dioxetane and fluorescent substance such as 4-methylumbelliferyl phosphate via alkaline phosphatase, etc., bound to an anti-fluorescein antibody, biotin etc. for using fluorescent, luminescent, or coloring substances bound to avidin, or a donor fluorescent dye and an acceptor fluorescent dye for using fluorescence resonance energy transfer (FRET).

Preferably, the labeling substance is biotin, and labeling of the oligonucleotide is preferably performed by biotinylating the 5' end or 3' end. When the labeling substance is biotin, the substance specifically binding to the labeling substance is streptavidin or avidin.

More specifically describing a pair of "probes capable of self-aggregation", the first oligonucleotide is an oligonucleotide containing at least a nucleic acid region X, a nucleic acid region Y, and a nucleic acid region Z in order from the 5'-end side, and the second oligonucleotide is an oligonucleotide containing at least a nucleic acid region X' complementary to the nucleic acid region X, a nucleic acid region Y' complementary to the nucleic acid region Y, and a nucleic acid Z' complementary to the nucleic acid region Z in order from the 5'-end side.

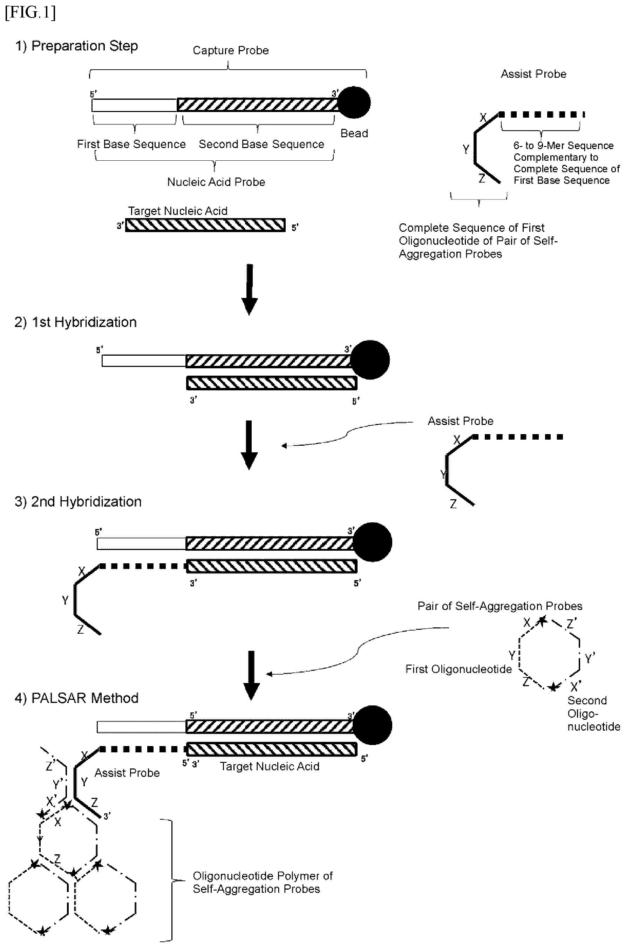

As the first aspect of the method for detecting a target nucleic acid of the present invention, a method for detecting by the PALSAR with the assist probe flanking to the target nucleic acid will specifically be described with reference to Fig. 1. First, a sample possibly containing the target nucleic acid, a capture probe, and an assist probe are prepared (1 of Fig. 1)). The capture probe of the present invention contains
(A) a nucleic acid probe, and
(B) a bead immobilized on a 3'-end nucleotide portion of the nucleic acid probe.

The nucleic acid probe contains a first base sequence and a second base sequence complementary to the complete sequence of the target nucleic acid.

The assist probe contains a 6- to 9-mer sequence capable of flanking to the 3' end of the target nucleic acid and complementary to the complete sequence of the first base sequence of the nucleic acid probe and a sequence of one of paired probes capable of self-aggregation.

First, the target nucleic acid and the capture probe are brought into contact (1st hybridization), and the assist probe is then brought into contact (2nd hybridization) (2 and 3 of Fig. 1).

Since the second base sequence of the nucleic acid probe is a sequence complementary to the complete sequence of the target oligonucleotide, these sequences are hybridized and bound, and since the assist probe is a sequence complementary to the complete sequence of the first base sequence of the nucleic acid probe, these sequences are hybridized and bound.

This step provides a hybridization product of the capture probe, the target nucleic acid, and the assist probe (3 of Fig. 1). Although these contact steps are separately performed in an example shown in Fig. 1, these steps may be performed at the same time. Specifically, the target nucleic acid, the capture probe, and the assist probe can be brought into contact at almost the same time to obtain the hybridization product of three components without separating the 1st hybridization and the 2nd hybridization.

When the target nucleic acid does not exist, since the 5' end of the assist probe of the present invention has no flanking target nucleic acid and has only 6 to 9 bases of the sequence complementary to the first base sequence, the probe cannot hybridize with the region of the first base sequence and, even if hybridizes, the probe is immediately detached. Therefore, capturing by the capture probe is ensured in the presence of the target nucleic acid.

A pair of probes capable of self-aggregation composed of the first and second oligonucleotides is brought into contact with a sample containing the hybridization product to form a complex between the hybridization product and the oligonucleotide polymer formed by self-aggregation of the first and second oligonucleotides (so-called PALSAR reaction, 4 of Fig. 1).

In the pair of probes capable of self-aggregation composed of the first and second oligonucleotides, the first oligonucleotide is an oligonucleotide containing the nucleic acid region X, the nucleic acid region Y, and the nucleic acid region Z in order from the 5'-end side, the second oligonucleotide is an oligonucleotide containing the nucleic acid region X', the nucleic acid region Y', and the nucleic acid region Z' in order from the 5'-end side, and X, Y, and Z are complementary to X', Y', and Z', respectively (hereinafter, the nucleic acid region may be simply referred to as X, Y, Z, X', Y', Z'). Additionally, labeling substances (stars in 4 of Fig. 1) are attached to the 5' ends of the first and second oligonucleotides.

The Y' and Z' of the second oligonucleotide hybridize with the Y and Z of the assist probe of the hybridization product, and the X', Y', and Z' of the second oligonucleotide hybridize with the X, Y, and Z of the first oligonucleotide, respectively. The X and X', the Y and Y', and the Z and Z' repeat hybridization one after another to form an oligonucleotide polymer of the self-aggregation probe (4 of Fig. 1).

The complex between the hybridization product of the capture probe, the target nucleic acid, and the assist probe and the self-aggregated first and second oligonucleotides is separated through precipitation by centrifugation etc., and the concentration of the target nucleic acid etc. can be measured by detecting the labeling substance etc. of the first and second oligonucleotides.

The target nucleic acid can also be detected by detecting the hybridization product without performing the PALSAR reaction.

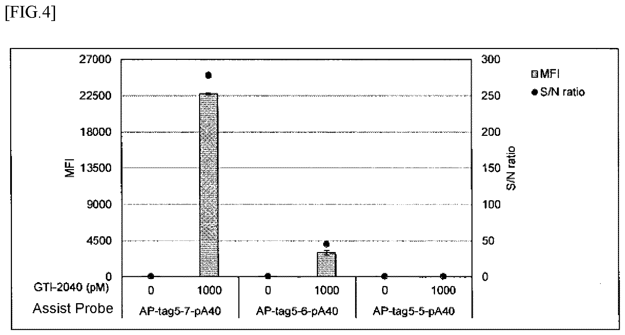

A specific example of the PALSAR method is described in Figs. 4 to 14 of WO 2013/172305, which can be appropriately modified based on the common knowledge of those skilled in the art using a probe for forming a dimer etc. and applied to the self-aggregating reaction of the present invention.

The complex between the hybridization product of the capture probe, the target nucleic acid, and the assist probe and the self-aggregated first and second oligonucleotides of the present invention is preferably separated, and the method for separating the complex is not particularly limited, and preferred examples include a centrifugation method and a suction filtration method.

A step of precipitating the capture probe and the complex thereof by "centrifugation" is usually performed by centrifugation at 500 to 3000 ×g for 0.2 to 5 minutes at 20 to 30 °C, centrifugation at 800 to 1500 ×g for 0.5 to 2 minutes at 23 to 28 °C, preferably centrifugation at 1000 ×g for 1 minute at 25 °C. More specifically, an instruction manual of the manufacturer of beads may be followed.

The step of separating the capture probe and the complex thereof by the "suction filtration method" can be performed by the method described in Biochem Biophys Res Commun. 2015 Nov 27; 467(4): 1012-8. Specifically, a solution containing the capture probe and the complex thereof is transferred to a filter plate and the step is usually performed by suction in a negative pressure range of 1 to 10 in.Hg at 20 to 30 °C, suction at 1 to 10 in.Hg at 23 to 28 °C, preferably suction at 1 to 5 in.Hg at 25 °C. A suction time may be set such that a liquid is visually removed from on the filter, and examples thereof include 1 second to 5 minutes, preferably 5 seconds to 1 minute. The pore size of the filter plate is preferably smaller than the diameter of the capture probe, and is preferably 1.2 µm or 1.0 µm, for example. Examples of specific members include filter plate: MultiScreen^{(registered trademark)} HTS-BV plate (Merck Millipore, product number: MSBVN1250), manifold: MultiScreen^{(registered trademark)} HTS Vacuum Manifold (Merck Millipore), suction pump: Chemical duty pump (Merck Millipore, catalog number: WP6110060).

### (Sample)

The "sample" used in the method of the present invention is body fluid such as whole blood, serum, plasma, lymph, urine, saliva, tears, sweat, gastric fluid, pancreatic fluid, bile, pleural fluid, joint cavity fluid, cerebrospinal fluid, spinal fluid, and bone marrow fluid, or tissues of liver, kidney, lung, heart, etc. of human, monkey, dog, pig, rat, guinea pig, or mouse. Preferably, the sample is human whole blood, serum, plasma, or urine. More preferably, the sample is whole blood, serum, plasma, or urine of a human to which a medicine containing the target nucleic acid is administered. These may be diluted with water or a buffer solution before use. The sample of the present invention also includes a target nucleic acid having a known concentration diluted with water or a buffer solution.

The buffer solution may be any commonly used buffer and examples thereof include Tris-hydrogen, boric acid, phosphoric acid, acetic acid, citric acid, succinic acid, phthalic acid, glutaric acid, maleic acid, glycine, and salts thereof, as well as Good's buffers such as MES, Bis-Tris, ADA, PIPES, ACES, MOPSO, BES, MOPS, TES, and HEPES, and examples of water include RNase and DNase free water.

Examples of the method for detecting the oligonucleotide polymer formed by self-aggregation of the first and second oligonucleotides of the present invention include a turbidity method, an agarose gel electrophoresis method, an absorbance method, a fluorescence measurement method, an electrochemical luminescence method, and a flow cytometry method, and the flow cytometry method is preferable.

The flow cytometry method is performed by, for example, allowing the reaction solution after the self-aggregation reaction (PALSAR reaction) to flow into a flow path of a flow cytometer, making an adjustment such that the complexes ([capture probe]-[target nucleic acid]-[assist probe]-[oligonucleotide polymer]) pass through the light source one by one, and detecting a first fluorescence emitted by a first fluorescent substance and a second fluorescence emitted by a second fluorescent substance when the individual complex passes through. "Detecting the first fluorescence emitted by the first fluorescent substance and the second fluorescence emitted by the second fluorescent substance" preferably means that the first fluorescence emitted by the first fluorescent substance and the second fluorescence emitted by the second fluorescent substance are detected at the same time or at the same opportunity. As used herein, "detecting at the same time or at the same opportunity" means that the first fluorescent substance and the second fluorescent substance contained in one complex (complex of multiple components) are simultaneously or sequentially excited in the flow path of the flow cytometer, and the emitted first and second fluorescences are detected as the fluorescences emitted from the one complex.

More specifically, examples of the first fluorescent substance include fluorescent substances contained in the solid phase of the capture probe, and examples of the second fluorescent substance include fluorescent substance previously bound to a substance that specifically binds to the labeling substance attached to the first and second oligonucleotides. In this way, the oligonucleotide polymer can be detected by detecting the first fluorescence emitted by the first fluorescent substance and the second fluorescence emitted by the second fluorescent substance by using the flow cytometry method.

### (Detection kit)

In the present invention, a kit for detecting the target nucleic acid in the sample includes at least the following constituent elements:
(1) a capture probe for capturing a target nucleic acid,
   the capture probe containing
   (A) a nucleic acid probe, and
   (B) a solid phase or an adapter or linker flanking to a 3'-end or 5'-end nucleotide of the nucleic acid probe,
      the nucleic acid probe containing:
      a first base sequence; and
      a second base sequence complementary to a complete or partial sequence of the target nucleic acid;
(2) a pair of probes capable of self-aggregation consisting of first and second oligonucleotides; and
(3) an assist probe containing a 6- to 9-mer sequence capable of flanking to the target nucleic acid and complementary to a complete or partial sequence of the first base sequence of the nucleic acid probe, and a complete or partial sequence of at least one of the first and second oligonucleotides.

The constituent elements are as described above.

Specific forms will hereinafter be described with examples to facilitate the understanding of the present invention; however, the present invention is not limited to these examples.

### EXAMPLES

### [Example 1]

### 1. Materials and Methods

### (1) Target Nucleic Acid

The oligonucleotide GTI-2040 used by Wei et al. was used as the target nucleic acid to be measured (see Reference 1). The sequence of GTI-2040 is 5'-GGCTAAATCGCTCCACCAAG-3'(SEQ ID NO:1). The synthesis of the oligonucleotide was commissioned to Nihon Gene Research Laboratories, Inc. (HPLC purification grade). The target nucleic acid is completely phosphorothioated as in Wei et al. GTI-2040 was prepared to 1,000 pM by using TE (10 mM Tris-HCl (pH 8.0), 1 mM EDTA (pH 8.0)) before use. A blank sample containing no target nucleic acid was also prepared.

### (Reference Document 1)

Wei X, Dai G, Marcucci G, Liu Z, Hoyt D, Blum W, Chan KK. A specific picomolar hybridization-based ELISA assay for the determination of phosphorothioate oligonucleotides in plasma and cellular matrices. Pharm Res. 2006 Jun;23(6): 1251-64.

### (2) Preparation of Capture Probe

A probe (referred to as a capture probe) was prepared by binding an oligonucleotide probe (nucleic acid probe) consisting of a base sequence complementary to GTI-2040 (20 bases, the second base sequence) and an arbitrary base sequence (10 bases, the first base sequence) to Luminex's MicroPlex^{™} Microspheres (Region No. 56, product number: LC10056-01) via NH₂ modification at the 3' end. The binding method was in accordance with the Luminex manual collection (xMAP^{(registered trademark)} Cookbook, 4th Edition). The following GTI-2040-1A CP was used as the oligonucleotide probe (nucleic acid probe) consisting of a base sequence (20 bases) complementary to GTI-2040 and an arbitrary base sequence (10 bases).
<base sequence of GTI-2040-1A CP>
5'-ATAACTAGTGCTTGGTGGAGCGATTTAGCC-(NH₂)-3' (base portion is SEQ ID NO:2)

### (3) Capture of Target Nucleic Acid by Capture Probe (1st Hybridization)

To 10 µL of the target nucleic acid, 10 µL of a 1st Hybridization reaction solution was added to make a total of 20 µL, and the reaction was performed at 45 °C for 1 hour.

### (3-1) Composition of 1st Hybridization Reaction Solution

Capture probe prepared in (2): 0.25 µL (500 pieces), 5M TMAC (tetramethylammonium chloride): 6 µL, 10× supplement [500 mM Tris-HCl (pH 8.0), 40 mM EDTA (pH 8.0), 8% N-Lauroyl sarcosine sodium]: 3.2 µL, 1 pmol/µL assist probe: 0.04 µL, Nuclease-Free Water: 0.51 µL

### (3-2) 1st Hybridization Reaction

As the assist probe used in Example 1, an assist probe (AP1-n1, API, or API + n1) consisting of a base sequence (8, 9, or 10 bases) complementary to a portion or all of the first base sequence of the nucleic acid probe and the same base sequence (39 bases) as a signal amplification probe (HCP-1) was used.

### <Base sequence of AP1-n1>

5'-CACTAGTTCAACAATCAGGACGATACCGATGAAGGATATAAGGAGTG-(Biotin)-3' (base portion is SEQ ID NO:3)

### <Base sequence of AP1>

5'-CACTAGTTACAACAATCAGGACGATACCGATGAAGGATATAAGGAGTG-(Biotin)-3' (base portion is SEQ ID NO:4)

### <Base sequence of AP1+n1>

5'-CACTAGTTATCAACAATCAGGACGATACCGATGAAGGATATAAGGAGTG-(Biotin)-3' (base portion is SEQ ID NO:5)

### (4) Self-Aggregation Reaction (PALSAR Reaction)

To 20 µL of the reaction solution after the 1st Hybridization reaction, 10 µL of a PALSAR reaction solution was added to make a total of 30 µL, and the reaction was performed at 30 °C for 1 hour.

The sequences of the pair of probes capable of self-aggregation (also referred to as signal amplification probes) used in this example are the following HCP-1 and HCP-2 labeled with biotin at the 5' end.

### <Base sequence of HCP-1>

5'-(Biotin)-CAACAATCAGGACGATACCGATGAAGGATATAAGGAGTG-3' (base portion is SEQ ID NO:6)

### <Base sequence of HCP-2>

5'-(Biotin)-GTCCTGATTGTTGCTTCATCGGTATCCACTCCTTATATC-3' (base portion is SEQ ID NO:7)

### (4-1) Composition of PALSAR Reaction Solution

5M TMAC: 3 µL, 10× supplement [500 mM Tris-HCl (pH 8.0), 40 mM EDTA (pH 8.0), 8% N-lauroyl sarcosine sodium]: 1.6 µL, 20 pmol/µL HCP-1: 0.35 µL, 20 pmol/µL HCP-2: 0.35 µL, Nuclease-Free Water: 4.7 µL

### (5) Fluorescence Detection

The reaction solution after the self-aggregation reaction was washed twice with 1×PBS-TP [1×PBS [137mM Sodium Chloride, 8.1 mMDisodium Phosphate, 2.68 mM Potassium Chloride, 1.47 mM Potassium Dihydrogenphosphate], 0.02 % Tween20, 1.5 ppm ProClin300], 50 µL of a detection reagent [SAPE (Streptavidin-R-Phycoerythrin, manufactured by Prozyme): 5 µg/mL] was then added, and the mixture was allowed to stand for 10 minutes under shading at 25 °C, and then washed twice with 1×PBS-TP. Subsequently, 75 µL of 1×PBS-TP was added, and the fluorescence from beads and SAPE conjugate was measured with a Luminex System (manufactured by Luminex) to detect the signal of the target nucleic acid.

### (6) Results

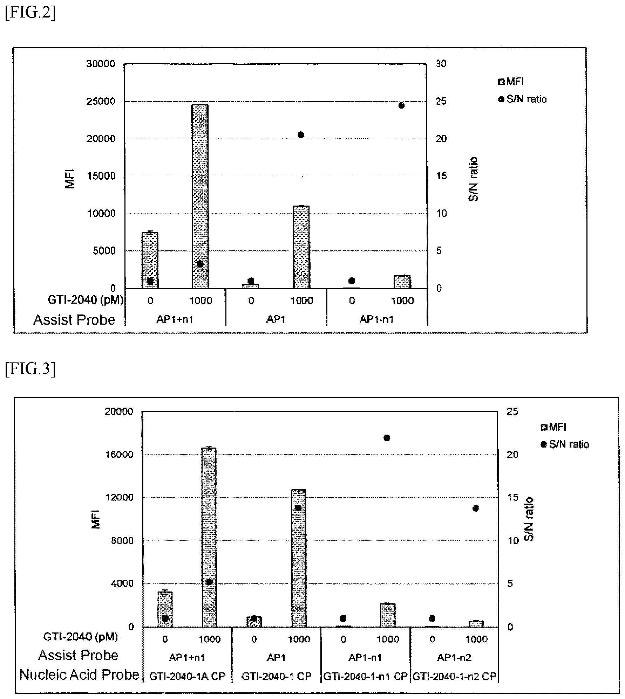

The measurement results are shown in Fig. 2. When the assist probe (AP1-n1, AP1) consisting of a base sequence complementary to the 8 or 9 bases of the first base sequence of the nucleic acid probe and the same base sequence as the signal amplification probe was used, the detection sensitivity to the target nucleic acid was excellent, and the S/N ratio was also high. On the other hand, when the assist probe (AP1+n1) consisting of a base sequence complementary to the 10 bases of the first base sequence of the nucleic acid probe and the same base sequence as the signal amplification probe is used, the background value was high, and the S/N ratio was low.

### [Example 2]

### 1. Materials and Methods

### (1) Target Nucleic Acid

GTI-2040 used in Example 1 was used as the target nucleic acid to be measured. GTI-2040 was prepared to 1,000 pM by using TE (10 mM Tris-HCl (pH 8.0), 1 mM EDTA (pH 8.0)) before use. A blank sample containing no target nucleic acid was also prepared.

### (2) Preparation of Capture Probe

A probe (referred to as a capture probe) was prepared by binding an oligonucleotide probe (nucleic acid probe) consisting of a base sequence complementary to GTI-2040 (20 bases, the second base sequence) and an arbitrary base sequence (7, 8, 9, or 10 bases, the first base sequence) to Luminex's MicroPlex^{™} Microspheres (Region No. 43, product number: LC10043-01, or Region No. 56, product number: LC10056-01) via NH₂ modification at the 3' end. The binding method was in accordance with the Luminex manual collection (xMAP^{(registered trademark)} Cookbook, 4th Edition).

GTI-2040-1-n2 CP, GTI-2040-1-n1 CP, GTI-2040-1 CP, and GTI-2040-1A CP were used as nucleic acid probes.

### <Base sequence of GTI-2040-1-n2 CP>

5'-ACTAGTGCTTGGTGGAGCGATTTAGCC-(NH₂)-3' (base portion is SEQ ID NO:8)

### <Base sequence of GTI-2040-1-n1 CP>

5'-AACTAGTGCTTGGTGGAGCGATTTAGCC-(NH₂)-3' (base portion is SEQ ID NO:9)

### <Base sequence of GTI-2040-1 CP>

5'-TAACTAGTGCTTGGTGGAGCGATTTAGCC-(NH₂)-3' (base portion is SEQ ID NO: 10)

### <Base sequence of GTI-2040-1A CP>

5'-ATAACTAGTGCTTGGTGGAGCGATTTAGCC-(NH₂)-3' (base portion is SEQ ID NO:2)

### (3) Capture of Target Nucleic Acid by Capture Probe (1st Hybridization)

To 10 µL of the target nucleic acid, 10 µL of the 1st Hybridization reaction solution was added to make a total of 20 µL, and the reaction was performed at 45 °C for 1 hour.

### (3-1) Composition of 1st Hybridization Reaction Solution

Capture probe prepared in (2): 0.25 µL (500 pieces), 5M TMAC: 6 µL, 10× supplement [500 mM Tris-HCl (pH 8.0), 40 mM EDTA (pH 8.0), 8% N-lauroyl sarcosine sodium]: 3.2 µL, 1 pmol/µL assist probe: 0.04 µL, Nuclease-Free Water: 0.51 µL

### (3-2) 1st Hybridization Reaction

As the assist probe used in Example 2, the assist probe (AP1-n2, AP1-n1, API, or AP1+n1) consisting of a base sequence (7, 8, 9, or 10 bases) complementary to all of the first base sequence of the nucleic acid probe and the same base sequence (39 bases) as the signal amplification probe (HCP-1) was used.

### <Base sequence of AP1-n2>

5'-CACTAGTCAACAATCAGGACGATACCGATGAAGGATATAAGGAGTG-(Biotin)-3' (base portion is SEQ ID NO: 11)

### <Base sequence of AP1-n1>

5'-CACTAGTTCAACAATCAGGACGATACCGATGAAGGATATAAGGAGTG-(Biotin)-3' (base portion is SEQ ID NO:3)

### <base sequence of AP1>

5'-CACTAGTTACAACAATCAGGACGATACCGATGAAGGATATAAGGAGTG-(Biotin)-3' (base portion is SEQ ID NO:4)

### <Base sequence of AP1+n1>

5'-CACTAGTTATCAACAATCAGGACGATACCGATGAAGGATATAAGGAGTG-(Biotin)-3' (base portion is SEQ ID NO:5)

### (4) Self-Aggregation Reaction (PALSAR)

To 20 µL of the reaction solution after the 1st Hybridization reaction, 10 µL of a PALSAR reaction solution was added to make a total of 30 µL, and the reaction was performed at 30 °C for 1 hour. The same probes as in Example 1 were used for the signal amplification probes.

### (4-1) Composition of PALSAR Reaction Solution

5M TMAC: 3 µL, 10× supplement [500 mM Tris-HCl (pH 8.0), 40 mM EDTA (pH 8.0), 8% N-lauroyl sarcosine sodium]: 1.6 µL, 20 pmol/µLHCP-1: 0.35 µL, 20 pmol/µL HCP-2: 0.35 µL, Nuclease-Free Water: 4.7 µL

### (5) Fluorescence Detection

The reaction solution after the self-aggregation reaction was washed twice with 1×PBS-TP [1×PBS [137mM Sodium Chloride, 8.1 mM Disodium Phosphate, 2.68 mM Potassium Chloride, 1.47 mM Potassium Dihydrogenphosphate], 0.02 % Tween20, 1.5 ppm ProClin300], 50 µL of the detection reagent [SAPE: 5 µg/mL] was then added, and the mixture was allowed to stand for 10 minutes under shading at 25 °C, and then washed twice with 1×PBS-TP. Subsequently, 75 µL of 1×PBS-TP was added, and the fluorescence from beads and SAPE conjugate was measured with a Luminex System (manufactured by Luminex) to detect the signal of the target nucleic acid.

### (6) Results

The measurement results are shown in Fig. 3. When the assist probe (AP1-n2, AP1-n1, API) consisting of a base sequence (7 to 9 bases) complementary to the first base sequence (7 to 9 bases) of the nucleic acid probe and the same base sequence as the signal amplification probe was used, the detection sensitivity to the target nucleic acid was excellent, and the S/N ratio was also high. On the other hand, when the assist probe (AP1+n1) consisting of a base sequence (10 bases) complementary to the first base sequence (10 bases) of the nucleic acid probe and the same base sequence as the signal amplification probe was used, the background value was high, and the S/N ratio was low.

### [Example 3]

### 1. Materials and Methods

### (1) Target nucleic acid

GTI-2040 used in Example 1 was used as the target nucleic acid to be measured. GTI-2040 was prepared to 1,000 pM by using TE (10 mM Tris-HCl (pH 8.0), 1 mM EDTA (pH 8.0)) before use. A blank sample containing no target nucleic acid was also prepared.

### (2) Preparation of Capture Probe

A probe (referred to as a capture probe) was prepared by binding an oligonucleotide probe (nucleic acid probe) consisting of a base sequence complementary to GTI-2040 (20 bases, the second base sequence) and an arbitrary base sequence (10 bases, the first base sequence) to Luminex's MicroPlex^{™} Microspheres (Region No. 37, product number: LC10037-01) via NH₂ modification at the 3' end. The binding method was in accordance with the Luminex manual collection (xMAP^{(registered trademark)} Cookbook, 4th Edition).

GTI-2040-tag5 CP was used as the nucleic acid probe.

### <Base sequence of GTI-2040-tag5 CP>

5'-TGGAGGAACGCTTGGTGGAGCGATTTAGCC-(NH₂)-3' (base portion is SEQ ID NO: 12)

### (3) Capture of Target Nucleic Acid by Capture Probe (1st Hybridization)

To 10 µL of the target nucleic acid, 10 µL of the 1st Hybridization reaction solution was added to make a total of 20 µL, and the reaction was performed at 45 °C for 1 hour.

### (3-1) Composition of 1st Hybridization Reaction Solution

Capture probe prepared in (2): 0.25 µL (500 pieces), 5M TMAC: 6 µL, 10× supplement [500 mM Tris-HCl (pH 8.0), 40 mM EDTA (pH 8.0), 8% N-lauroyl sarcosine sodium]: 3.2 µL, 0.1 pmol/µL assist probe: 0.4 µL, Nuclease-Free Water: 0.15 µL

### (3-2) 1st Hybridization Reaction

As the assist probe used in Example 3, an assist probe (AP-tag5-5-pA40, AP-tag5-6-pA40, or AP-tag5-7-pA40) consisting of a base sequence (5, 6, or 7 bases) complementary to a portion of the first base sequence of the nucleic acid probe and the same base sequence (40 bases) as a portion of the signal amplification probe (HCP-4).

### <Base sequence of AP-tag5-5-pA40>

5 '- CGTTCAAAAAAAAAAAAAAAAAAAAAAAAAAAAAAAAAAAAAAAA-3' (SEQ ID NO: 13)

### <Base sequence of AP-tag5-6-pA40>

5 '- CGTTCCAAAAAAAAAAAAAAAAAAAAAAAAAAAAAAAAAAAAAAAA-3' (SEQ ID NO:14)

### <Base sequence of AP-tag5-7-pA40>

5 '- CGTTCCTAAAAAAAAAAAAAAAAAAAAAAAAAAAAAAAAAAA-

3' (SEQ ID NO:15)

### (4) Self-Aggregation Reaction (PALSAR Reaction)

To 20 µL of the reaction solution after the 1st Hybridization reaction, 10 µL of a PALSAR reaction solution was added to make a total of 30 µL, and the reaction was performed at 30 °C for 1 hour.

The sequences of the signal amplification probes used in this example are the following HCP-3 and HCP-4 labeled with biotin at the 5' end.

### <Base sequence of HCP-3>

5'-(Biotin)-CAACAATCAGGACGATACCGATGAAGTTTTTTTTTTTTTTTTTTTT-3' (base portion is SEQ ID NO:16)

### <Base sequence of HCP-4>

5'-(Biotin)-GTCCTGATTGTTGCTTCATCGGTATCAAAAAAAAAAAAAAAAAAAA-3' (base portion is SEQ ID NO: 17)

### (4-1) Composition of PALSAR Reaction Solution

5M TMAC: 3 µL, 10× supplement [500 mM Tris-HCl (pH 8.0), 40 mM EDTA (pH 8.0), 8% N-lauroyl sarcosine sodium]: 1.6 µL, 20 pmol/µL HCP-3: 0.35 µL, 20 pmol/µL HCP-4: 0.35 µL, Nuclease-Free Water: 4.7 µL

### (5) Fluorescence Detection

The reaction solution after the self-aggregation reaction was washed twice with 1×PBS-TP [1×PBS [137mM Sodium Chloride, 8.1 mMDisodium Phosphate, 2.68 mM Potassium Chloride, 1.47 mM Potassium Dihydrogenphosphate], 0.02 % Tween20, 1.5 ppm ProClin300], 50 µL of the detection reagent [SAPE: 5 µg/mL] was then added, and the mixture was allowed to stand for 10 minutes under shading at 25 °C, and then washed twice with 1×PBS-TP. Subsequently, 75 µL of 1×PBS-TP was added, and the fluorescence from beads and SAPE conjugate was measured with a Luminex System (manufactured by Luminex) to detect the signal of the target nucleic acid.

### (6) Results

The measurement results are shown in Fig. 4. When the assist probe (AP-tag5-7-pA40, AP-tag5-6-pA40) consisting of a base sequence complementary to 7 or 6 bases of the first base sequence of the nucleic acid probe and the same base sequence as a portion of the signal amplification probe was used, the detection sensitivity to the target nucleic acid was excellent and the S/N ratio was also high. On the other hand, when the assist probe (AP-tag5-5-pA40) consisting of a base sequence complementary to 5 bases of the first base sequence of the nucleic acid probe and the same base sequence as a portion of the signal amplification probe was used, the detection sensitivity was almost zero and could not be measured.

From Examples 1 to 3, it was confirmed that the assist probe contains 6- to 9-mer sequence capable of flanking to the end of the target nucleic acid and complementary to a complete or partial sequence of the first base sequence of the nucleic acid probe and therefore provides the high detection sensitivity and the high S/N ratio.

### INDUSTRIAL APPLICABILITY

According to the present invention, a more sensitive detection method and quantification method can be provided in the method for detecting a target nucleic acid using the PALSAR method. The method for detecting or quantifying a target nucleic acid of the present invention can be applied to measurement of drug concentration in a biological sample of an animal or a human to which a drug is administered in pharmacokinetic/pharmacodynamic (PK/PD) screening tests in an exploratory stage of drug development, safety tests, pharmacological tests, and pharmacokinetic tests in a

## Claims

1. A method for recovering a target nucleic acid in a sample, comprising:
(i) a step of bringing a target nucleic acid in a sample, a capture probe, and an assist probe into contact with each other for hybridization,
the capture probe containing
(A) a nucleic acid probe, and
(B) a solid phase or an adapter or linker flanking to a 3'-end or 5'-end nucleotide of the nucleic acid probe,
the nucleic acid probe containing:
a first base sequence; and
a second base sequence complementary to a complete or partial sequence of the target nucleic acid,
the assist probe containing a 6- to 9-mer sequence capable of flanking to the target nucleic acid and complementary to a complete or partial sequence of the first base sequence of the nucleic acid probe; and
(ii) a step of recovering a hybridization product contained in the sample.

2. A method for detecting a target nucleic acid in a sample, comprising:
(i) a step of bringing a target nucleic acid in a sample, a capture probe, and an assist probe into contact with each other for hybridization,
the capture probe containing
(A) a nucleic acid probe, and
(B) a solid phase or an adapter or linker flanking to a 3'-end or 5'-end nucleotide of the nucleic acid probe,
the nucleic acid probe containing:
a first base sequence; and
a second base sequence complementary to a complete or partial sequence of the target nucleic acid,
the assist probe containing a 6- to 9-mer sequence capable of flanking to the target nucleic acid and complementary to a complete or partial sequence of the first base sequence of the nucleic acid probe;
(ii) a step of recovering a hybridization product contained in the sample; and
(iii) a step of detecting the recovered hybridization product.

3. A method for detecting a target nucleic acid in a sample by forming a complex with a pair of probes capable of self-aggregation consisting of first and second oligonucleotides, comprising:
(i) a step of bringing a target nucleic acid in a sample, a capture probe, and an assist probe into contact with each other for hybridization,
the capture probe containing
(A) a nucleic acid probe, and
(B) a solid phase or an adapter or linker flanking to a 3'-end or 5'-end nucleotide of the nucleic acid probe,
the nucleic acid probe containing:
a first base sequence; and
a second base sequence complementary to a complete or partial sequence of the target nucleic acid,
the assist probe containing a 6- to 9-mer sequence capable of flanking to the target nucleic acid and complementary to a complete or partial sequence of the first base sequence of the nucleic acid probe, and a complete or partial sequence of at least one of the first and second oligonucleotides;
(ii) a step of bringing the first and second oligonucleotides into contact with the hybridization product to form a polymer complex between the hybridization product and an oligonucleotide polymer formed by self-aggregation of the first and second oligonucleotides; and
(iii) a step of detecting the polymer complex.

4. The method according to any one of claims 1 to 3, wherein the step of bringing a target nucleic acid in a sample, a capture probe, and an assist probe into contact with each other for hybridization includes two stages of hybridization steps, which are a first hybridization step of bringing the target nucleic acid into contact with the capture probe, and a second hybridization step of bringing a first hybridization product into contact with the assist probe.

5. The method according to claim 4, wherein the two stages of hybridization steps are steps performed at the same time.

6. The method of any of claims 3 to 5, wherein the assist probe contains a 6- to 9-mer sequence capable of flanking to the target nucleic acid and complementary to the complete sequence of the first base sequence of the nucleic acid probe, and a complete or partial sequence of at least one of the first and second oligonucleotides.

7. The method according to any one of claims 1 to 6, wherein the assist probe is capable of flanking to the end of the target nucleic acid.

8. The method according to any one of claims 1 to 7, wherein the sample is a blood-derived component.

9. The method according to any one of claims 1 to 8, wherein the target nucleic acid is a chemically modified nucleic acid.

10. The method according to claim 9, wherein the chemically modified nucleic acid is LNA, BNA, phosphorothioate oligo, morpholino oligo, boranophosphate oligo, 2'-0-methylated RNA (2'-OMe), 2'-O-methoxyethylated RNA (2'-MOE), or 2'-F-RNA.

11. A kit for detecting a target nucleic acid in a sample, comprising:
(1) a capture probe for capturing a target nucleic acid,
the capture probe containing
(A) a nucleic acid probe, and
(B) a solid phase or an adapter or linker flanking to a 3'-end or 5'-end nucleotide of the nucleic acid probe,
the nucleic acid probe containing:
a first base sequence; and
a second base sequence complementary to a complete or partial sequence of the target nucleic acid;
(2) a pair of probes capable of self-aggregation consisting of first and second oligonucleotides; and
(3) an assist probe containing a 6- to 9-mer sequence capable of flanking to the target nucleic acid and complementary to a complete or partial sequence of the first base sequence of the nucleic acid probe, and a complete or partial sequence of at least one of the first and second oligonucleotides.

12. The detection kit according to claim 11, wherein the assist probe contains a 6- to 9-mer sequence capable of flanking to the target nucleic acid and complementary to the complete sequence of the first base sequence of the nucleic acid probe, and a complete or partial sequence of at least one of the first and second oligonucleotides.

13. The detection kit according to claim 11 or 12, wherein the assist probe is capable of flanking to the end of the target nucleic acid.
